(19) **Europäisches Patentamt European Patent Office Office européen des brevets**

(11) **EP 4 595 975 A1**

(12) **EUROPEAN PATENT APPLICATION**

(43) Date of publication:
06.08.2025 Bulletin 2025/32

(21) Application number: 25153235.4

(22) Date of filing: **22.01.2025**

(51) International Patent Classification (IPC):
**A61K 39/00** (2006.01)  **A61P 35/00** (2006.01)
**C07K 7/06** (2006.01)  **C07K 14/21** (2006.01)

(52) Cooperative Patent Classification (CPC):
**A61K 39/001169; A61P 35/00; C07K 7/06;
C07K 14/21;** A61K 2039/572; A61K 2039/575;
A61K 2039/6031; C07K 2319/04; C07K 2319/55;
C07K 2319/74

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **22.01.2024 US 202463623781 P**

(71) Applicant: **Taivital Biopharmaceutical Co. Ltd
Taoyuan City 320 (TW)**

(72) Inventors:
- LIN, Yipei
  **320 TAOYUAN CITY (TW)**
- HWANG, Jaulang
  **320 TAOYUAN CITY (TW)**

(74) Representative: **Lavoix
Bayerstraße 83
80335 München (DE)**

Remarks:
The complete document including Reference
Table(s) and the Sequence Listing(s) can be
downloaded from the EPO website

(54) **FUSION PROTEIN FOR INDUCING IMMUNE RESPONSE AND TREATING CANCER**

(57)    The present disclosure relates to fusion proteins for inducing immune response and treating cancer, and particularly to fusion proteins for use as an immunogenic enhancer for inducing humoral immune response and cell-mediated immune response.

EP 4 595 975 A1

**Description**

RELATED APPLICATIONS

This application claims priority to U.S. Provisional Application Ser. No. 63/623,781, filed January 22, 2024, the content of which is incorporated herein by reference in its entirety.

SEQUENCE LISTING

The instant application contains a Sequence Listing submitted electronically in .xml format and hereby incorporated by reference in its entirety. The .xml copy, created on 9 January 2025, is named "US15429-Sequence Listing.xml" and is 11 kilobytes in size.

FIELD OF THE INVENTION

The present disclosure relates to fusion proteins for inducing immune response and treating cancer, and particularly to fusion proteins for use as an immunogenic enhancer for inducing humoral immune response and cell-mediated immune response.

BACKGROUND

Conventional chemical and physical therapies for cancer are known to have limitations, including tumor recurrence and development of resistance in cancer cells. These issues can lead to increased mortality rates and treatment failure, mainly from non-specific toxicity thereof and overexpression of ATP-binding cassette (ABC) transporter proteins by tumor cells. The ABC transporter proteins play a role in expelling chemotherapeutic agents from the cell cytosol to the extracellular space, making it difficult for the drugs to effectively target the tumor cells.

Surgical removal of solid tumors, however, has been associated with increased survival rates, despite often resulting in permanent scarring and disfigurement of organs and tissue. Additionally, there is a risk of unidentified micrometastases, small clusters of tumor cells that may remain after surgery and can contribute to cancer relapse.

Targeted cancer therapies are a type of immune therapy that focuses on blocking specific receptors expressed by cancer cells or delivering therapeutic agents or toxins directly to cancer sites. The latter approach involves the use of conjugate proteins that consist of site-specific domains, such as monoclonal antibodies, Fab, Fv, and receptor ligands, along with cytotoxic effector molecules derived from bacteria, plants, animals, or insects. These conjugates are referred to as immunotoxins (ITs). The development of ITs was inspired by the concept of a "magic bullet," wherein a lethal agent is chemically or genetically fused with a site-specific moiety to ensure specificity towards cancer cells. In IT constructions, either bacterial or plant toxins may be used. These toxins are capable of inducing apoptosis and halt protein synthesis machinery due to their ADP-ribosyl transferase activity that blocks peptide chain elongation.

There remains a need for improved cancer therapies with better clinical outcomes and reduced adverse effects.

SUMMARY OF THE INVENTION

In some aspects, the disclosure provides a fusion protein. The fusion protein includes an antigen-presenting cell (APC)-binding peptide or a CD91 receptor-binding peptide, a translocation peptide, a linker peptide, and one or more repeats of cysteine-rich peptide. In one embodiment, the fusion protein can further comprise a retention signaling domain selected from an endoplasmic reticulum (ER) retention sequence, a Golgi retention sequence and a proteosome localizing sequence.

In one embodiment, the fusion protein includes in sequential order from the N-terminus to the C-terminus: the APC-binding peptide or the CD91 receptor-binding peptide, the translocation peptide, the linker peptide, the one or more repeats of cysteine-rich peptide, and optionally the retention signaling domain.

In some embodiments, the APC-binding peptide or the CD91 receptor-binding peptide has an amino acid sequence having at least 90% or at least 95% identity to SEQ ID NO: 1. In some embodiments, the APC-binding peptide or the CD91 receptor-binding peptide has an amino acid sequence having about 90%, about 91%, about 92%, about 93%, about 94%, about 95%, about 96%, about 97%, about 98%, about 99%, or about 100% identity to SEQ ID NO: 1. In one embodiment, the APC-binding peptide or the CD91 receptor-binding peptide has the amino acid sequence of SEQ ID NO: 1.

In some embodiments, the translocation peptide has an amino acid sequence having at least 90% or at least 95% identity to SEQ ID NO: 2. In some embodiments, the translocation peptide has an amino acid sequence having about 90%, about 91%, about 92%, about 93%, about 94%, about 95%, about 96%, about 97%, about 98%, about 99%, or about 100% identity to SEQ ID NO: 2. In one embodiment, the translocation peptide has the amino acid sequence of SEQ ID NO: 2.

In some embodiments, the cysteine-rich peptide includes an amino acid sequence having at least 60%, at least 65%, at least 70%, at least 75%, at least 80%, at least 85%, at least 90% or at least 95% identity to the amino acid sequence of PCCGCCGCGC (SEQ ID NO: 4). In some embodiments, the cysteine-rich peptide includes an amino acid sequence having about 60%, about 65%, about 70%, about 75%, about 80%, about 85%, about 90% (about 91%, about 92%, about 93%, about 94%, about 95%, about 96%, about 97%, about 98% , about 99%), or about 100% identity to the amino acid sequence of PCCGCCGCGC (SEQ ID NO: 4). In some embodiments, the cysteine-rich peptide includes an amino acid sequence that differs in one or more amino acid residues from the amino acid sequence of PCCGCCGCGC (SEQ ID NO: 4). In some embodiments, the cysteine-rich peptide includes an amino acid sequence that differs in one, two, three, four, five, or six amino acid residues from the amino acid sequence of PCCGCCGCGC (SEQ ID NO: 4). In one embodiment, the cysteine-rich peptide includes the amino acid sequence of PCCGCCGCGC (SEQ ID NO: 4).

In some embodiments, the cysteine-rich peptide includes 2 to 15 repeats of the amino acid sequence of PCCGCCGCGC (SEQ ID NO: 4). In some embodiments, the cysteine-rich peptide includes 2, 3, 4, 5, 6, 7, 8, 9, 10, 11, 12, 13, 14, or 15 repeats of the amino acid sequence of PCCGCCGCGC (SEQ ID NO: 4). In some embodiments, the cysteine-rich peptide includes or consists of 7 repeats of the amino acid sequence of PCCGCCGCGC (SEQ ID NO: 4), namely (PCCGCCGCGC)$_7$ (SEQ ID NO: 5).

In some embodiments, the cysteine-rich peptide carries an antigen that is linked to at least one cysteine residue in the cysteine-rich peptide. In some embodiments in which the cysteine-rich peptide includes the amino acid sequence of PCCGCCGCGC (SEQ ID NO: 4), one or more antigens is linked to one or more cysteine residues in the cysteine-rich peptide. For instance, one, two, three, four, five, or six antigens may be respectively linked to one, two, three, four, five, or six cysteine residues in the cysteine-rich peptide. In some embodiments, the antigens are the same or different among others.

Any antigen, in particular one with low immunogenicity, may be linked to multiple cysteine residues in the cysteine-rich peptide. Exemplary antigens include, but are not limited to, glycotopes (e.g., Tn, sTn, GM2, and GM3), glycolipids (e.g., Globo H and SSEA3), and glycophingolipids (e.g., B-I and B-II). The antigen can also be a steroid hormone, such as a glucocorticoid (e.g., prednisone, dexamethasone, or triamcinolone), a mineralocorticoid (e.g., fludrocortisone), a vitamin D (e.g., dihydrotachysterol), an androgen (e.g., oxandrolone or nandrolone), an estrogen (e.g., diethylstilbestrol), or a progestins (e.g., norethindrone or medroxyprogesterone acetate). Androgens and estrogens taken together are known as sex hormones.

In some embodiments, the antigen is a glycoantigen. In some embodiments, the antigen is a cancer-associated antigen. In some embodiments, the antigen includes one or more antigens selected from the group consisting of T, Tn, sialyl-Tn (sTn), GM2, GM3, phosphoserine, phosphothreonine, sialic acid, GlcNAc, Globo H, Lewis x, Lewis y, and a steroid hormone.

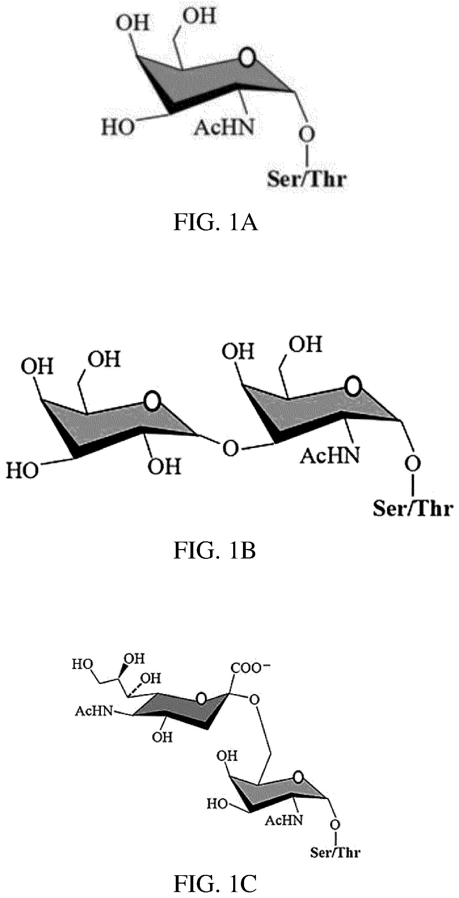

In one embodiment, the antigen includes one or more cancer-associated antigens selected from Tn antigen (GalNAcα1-O-Ser/Thr), T antigen (Galβ1-3GalNAcα1-O-Ser/Thr), sialyl-Tn (sTn) antigen, or a combination thereof. The structures of the Tn antigen, the T antigen, and the sTn antigen are shown in FIGs. 1A to 1C, respectively.

In some embodiments, the antigen is linked to the cysteine residue in the cysteine-rich peptide via a linker, such as a maleimide linker capable of reacting with both an amine group and a thio group. Examples of the maleimide linker may be N-ε-Maleimidocaproic acid, m-Maleimidobenzoyl-N-hydroxysuccinimide ester (MBS), N-ε-Maleimidocaproyl-oxysuccinimide ester (EMCS), or succinimidyl-6-[β-maleimidopropionamido]-hexanoate (SMPH).

In some embodiments, the fusion protein recited herein further includes a linker peptide between translocation peptide and the cysteine-rich peptide. The linker peptide has an amino acid sequence having at least 90% or at least 95% identity to SEQ ID NO: 3. In some embodiments, the linker peptide has an amino acid sequence having about 90%, about 91%, about 92%, about 93%, about 94%, about 95%, about 96%, about 97%, about 98%, about 99%, or about 100% identity to SEQ ID NO: 3. In one embodiment, the linker peptide has the amino acid sequence of SEQ ID NO: 3.

In some embodiments, the retention signaling domain includes an amino acid sequence chosen from KDEL, KKMP, KKTN, XDEL, XXEL or KKXX, in which X can represent any amino acid. Examples of the amino acid sequence of the retention signaling domain include, but are not limited to, KDEL, HDEF, HDEL, RDEF, RDEL, WDEL, YDEL, HEEF, HEEL, KEEL, REEL, KAEL, KCEL, KFEL, KGEL, KHEL, KLEL, KNEL, KQEL, KREL, KSEL, KVEL, KWEL, KYEL, KEDL, KIEL, DKEL, FDEL, KDEF, KKEL, HADL, HAEL, HIEL, HNEL, HTEL, KTEL, HVEL, NDEL, QDEL, REDL, RDEL, RNEL, RTDL, RTEL, SDEL, TDEL, SKEL, REDLK. In certain embodiments, the ER retention sequence included in the fusion protein recited herein includes the amino acid sequence of KDEL (SEQ ID NO: 6), RDEL (SEQ ID NO: 7), or REDLK (SEQ ID NO: 8). In some embodiments, the ER retention sequence may be multiple repeats of the amino acid sequence of KDEL (SEQ ID NO: 6), RDEL (SEQ ID NO: 7), or REDLK (SEQ ID NO: 8). For example, the ER retention sequence may be 2, 3, or 4 repeats of the amino acid sequence of KDEL (SEQ ID NO: 6), RDEL (SEQ ID NO: 7), or REDLK (SEQ ID NO: 8). In some embodiments, the ER retention sequence consists of the amino acid sequence of KDEL (SEQ ID NO: 6), RDEL (SEQ ID NO: 7), or REDLK (SEQ ID NO: 8). In one embodiment, the ER retention sequence includes or consists of the amino acid sequence of REDLK (SEQ ID NO: 8).

In some embodiments, the fusion protein recited herein includes an amino acid sequence having at least 90% or at least 95% identity to SEQ ID NO: 9. In some embodiments, the fusion protein includes an amino acid sequence having about 90%, about 91%, about 92%, about 93%, about 94%, about 95%, about 96%, about 97%, about 98%, about 99%, or about 100% identity to SEQ ID NO: 9. In one embodiment, the fusion protein includes the amino acid sequence of SEQ ID NO: 9. In another embodiment, the fusion protein consists of the amino acid sequence of SEQ ID NO: 9.

In some embodiments, the fusion protein recited herein does not include the amino acid sequence of SEQ ID NO: 10, or a fragment thereof as follows.

GDVSFSTRGTQNWTVERLLQAHRQLEERGYVFVGYHGTFLEAAQSIVFGGVRARSQDL

DAIWRGFYIAGDPALAYGYAQDQEPDARGRIRNGALLRVYVPRSSLPGFYRTSLTLAAP

EAAGEVERLIGHPLPLRLDAITGPEEEGGRLETILGWPLAERTVVIPSAIPTDPRNVGGDL

DPSSIPDKEQAISALPDYASQPGKPPREDLK (SEQ ID NO: 10).

In some aspects, the disclosure also provides an isolated nucleic acid. The isolated nucleic acid encodes the aforementioned fusion protein.

In some aspects, the disclosure also provides a vector. The vector includes the aforementioned nucleic acid.

In some aspects, the disclosure also provides a host cell. The host cell includes the aforementioned isolated nucleic acid or vector.

In some aspects, the disclosure also provides a pharmaceutical composition. The pharmaceutical composition includes the aforementioned fusion protein and a pharmaceutically acceptable carrier.

In some aspects, the disclosure also provides a kit. The kit includes the fusion protein recited herein or pharmaceutical composition recited herein. In some embodiments, the kit further includes instructions for use.

In some aspects, the disclosure also provides a method of treating cancer. The method includes administering to a subject in need thereof an effective amount of the fusion protein recited herein or the pharmaceutical composition recited herein.

In some embodiments, the cancer may be any cancer expressing Tn antigen, sTn antigen, and/or T antigen.

In some embodiments, the cancer is selected from the group consisting of breast cancer, liver cancer, colorectal cancer, bladder cancer, cervical cancer, leukemia, lung cancer, central nervous system cancer, melanoma, ovarian cancer, gastric cancer, pancreatic cancer, head and neck cancer, renal cancer, and prostate cancer.

In some aspects, the disclosure also provides a method of treating viral infection in a subject in need thereof. The method includes administering to the subject an effective amount of the fusion protein recited herein or the pharmaceutical composition recited herein.

In some aspects, the disclosure also provides a method of inducing an immune response in a subject. The method includes administering to a subject in need thereof an effective amount of the fusion protein recited herein or the pharmaceutical composition recited herein.

In some embodiments, the immune response is adaptive immune response. In one embodiment, the immune response is humoral immune response. In one embodiment, the immune response is cell-mediated immune response. In one embodiment, the immune response is the combination of humoral immune response and cell-mediated immune response.

## BRIEF DESCRIPTION OF THE DRAWINGS

The subject application contains at least one drawing executed in color. Copies of this patent or patent application publication with color drawing(s) will be provided by the U.S. Patent Office upon request and payment of the necessary fee. The following drawings form part of the present specification and are included to further demonstrate certain aspects of the present disclosure, the inventions of which can be better understood by reference to one or more of these drawings in combination with the detailed description of specific embodiments presented herein. Exemplary embodiments are illustrated in referenced figures. It is intended that the embodiments and figures disclosed herein are to be considered illustrative rather than restrictive.

**FIGs. 1A to 1C** show the chemical structures of the Tn antigen, the T antigen, and the sTn antigen, respectively.

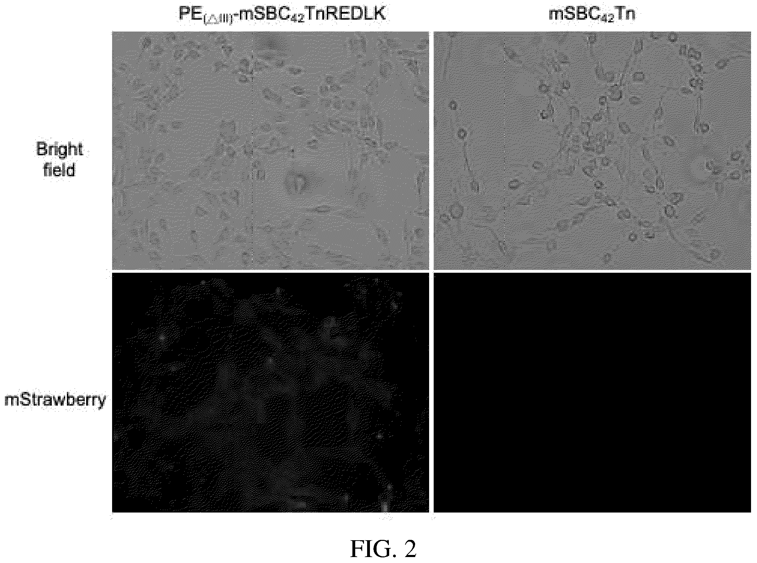

**FIG.** 2 shows internalization of the fusion protein, including *Pseudomonas* exotoxin (PE) A domains Ia, Ib, and II (PE (△III) and the RE retention sequence of REDLK, into NIH 3T3 cells. mStrawberry (mSB) red fluorescence was used as an indicator to confirm the internalization ability of the fusion protein.

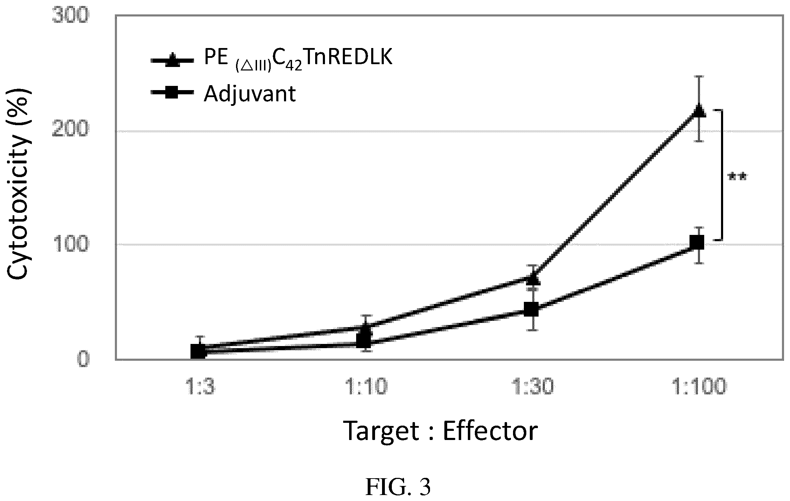

**FIG. 3** shows LDH release from MDA-MB-231 cells (target cells, T) expressing Tn antigen after co-culture with T cells (effector cells, E) that were isolated from the spleen of BALB/c mice immunized with the fusion protein, including *Pseudomonas* exotoxin (PE) A domains Ia, Ib, and II (PE (△III), Tn antigen, and the RE retention sequence of REDLK. T cells were co-cultured with MDA-MB-231 cells at different T: E ratios for 18 hr at 37°C. The amount of released LDH from damaged cells were detected to represent the ability of T cells to kill target cells. The culture with a T:E ratio of 1: 100 resulted in a significant cytotoxic effect, as indicated by the highest LDH release from MDA-MB-231 cells ($p$=0.004).

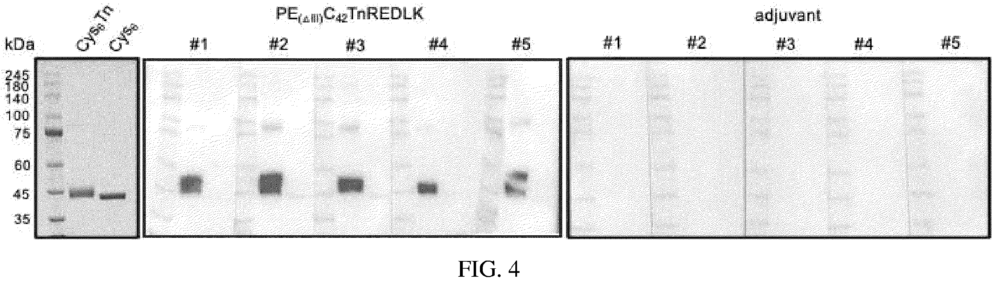

**FIG. 4** shows the immunization experiment monitoring antibody production from mice serum. Poly cysteine or poly cysteine conjugated with Tn were used as substrates to detect serum samples retrieved from mice immunized with the fusion protein, (PE (△III), Tn antigen, and REDLK sequence, or with an adjuvant alone.

## DETAILED DESCRIPTION

It is understood that the invention is not limited to the particular materials and methods recited herein. It is also to be understood that the terminology used herein is for the purpose of describing particular embodiments and is not intended to limit the scope of the present invention, which will be limited only by the appended claims.

### *Definitions*

It must be noted that, as used in this specification and the appended claims, the singular forms "a," "an," and "the" include plural referents unless the content clearly dictates otherwise. Thus, for example, reference to "a biomarker" includes a mixture of two or more biomarkers, and the like.

Throughout this specification, unless the context requires otherwise, the words "comprise", or variations such as "comprises" or "comprising", will be understood to imply the inclusion of a stated element or integer or group of elements or integers but not the exclusion of any other element or integer or group of elements or integers.

As used in this application, the terms "about" and "approximately" are used as equivalents. Any numerals used in this application with or without about/approximately are meant to cover any normal fluctuations appreciated by one of ordinary skill in the relevant art. In certain embodiments, the term "approximately" or "about" refers to a range of values that fall within 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

The terms "polynucleotide" and "nucleic acid," used interchangeably herein, refer to a polymeric form of nucleotides of more than about 100 nucleotides, either ribonucleotides or deoxyribonucleotides. Thus, this term includes, but is not limited to, single-, double-, or multi-stranded DNA or RNA, genomic DNA, cDNA, DNA-RNA hybrids, or a polymer comprising purine and pyrimidine bases or other natural, chemically or biochemically modified, non-natural, or derivatized nucleotide bases. "Oligonucleotide" generally refers to polynucleotides of between about 5 and about 100 nucleotides of single- or double-stranded DNA or RNA. However, for the purposes of this disclosure, there is no upper limit to the length of an oligonucleotide. Oligonucleotides are also known as "oligomers" or "oligos" and may be isolated from genes, or chemically synthesized by methods known in the art. The terms "polynucleotide" and "nucleic acid" should be understood to include, as applicable to the embodiments being recited, single-stranded (such as sense or antisense) and double-stranded polynucleotides.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

As used herein, the terms "treat," "treatment," and "treating" refer to an approach for obtaining beneficial or desired results, for example, clinical results. For the purposes of this disclosure, beneficial or desired results may include inhibiting or suppressing the initiation or progression of a disease; ameliorating, or reducing the development of, symptoms of a disease; or a combination thereof.

The term "sequence identity" refers to the percentage of bases or amino acids between two polynucleotide or polypeptide sequences that are the same, and in the same relative position. As such one polynucleotide or polypeptide sequence has a certain percentage of sequence identity compared to another polynucleotide or polypeptide sequence. For sequence comparison, typically one sequence acts as a reference sequence, to which test sequences are compared. The term "reference sequence" refers to a molecule to which a test sequence is compared.

As used herein, the term "effective amount" refers to the minimum amount of an agent or composition required to result in a particular physiological effect.

As interchangeably used herein, the terms "individual," "subject," "host," and "patient," refer to a mammal, including, but not limited to, murines (rats, mice), non-human primates, humans, canines, felines, ungulates (e.g., equines, bovines, ovines, porcines, caprines), etc. Particularly, the subject is vaccinated.

As used herein, the term "pharmaceutically acceptable" means being approved by a regulatory agency of a U.S. Federal or a state government or listed in the U.S. Pharmacopeia, European Pharmacopeia or other generally recognized pharmacopeia for use in mammals, and more particularly in humans.

*Fusion Protein*

The disclosure recited herein is based in part on the discovery that a fusion protein is capable of inducing strong immune response and is thus useful for treating cancer (e.g., cancers expressing Tn antigen, T antigen, and/or sTn antigen) or viral infection. The therapeutic benefit is due to internalization of the fusion protein into the target cell such as tumor cells followed by activation of both the MHC class I and MHC II antigen presentation pathway. In the MHC class I antigen presentation pathway, the fusion protein is broken down into smaller pieces of peptides by proteasomes, binds to class I MHC molecules in the endoplasmic reticulum (ER), and is then presented on the cell surface to CD8+ T cells, which in turn induces strong cell-mediated immunity. Cell-mediated immunity is beneficial to kill infected cells or tumor cells and is thus important for cancer and viral infection treatment. In the MHC II antigen presentation pathway, the fusion protein is degraded into small peptides in endolysosomes, and then the peptides bind with the class II MHC molecules to form a complex. The complex is transport to the cell surface of antigen-presenting cells (APC), allowing antigen presentation to CD4+ T cells and thus triggering humoral immune response. By triggering both the cell-mediated immune response and the humoral immune response, the fusion protein recited herein is effective in treating cancer or viral infection.

Accordingly, the fusion protein includes in sequential order from the N-terminus to the C-terminus: the APC-binding peptide or the CD91 receptor-binding peptide, the translocation peptide, the cysteine-rich peptide, and the ER retention sequence.

In one embodiment, an amino acid sequence having at least 90% or at least 95% identity to SEQ ID NO: 1 can be used as the APC-binding peptide or the CD91 receptor-binding peptide as recited herein. In one embodiment, an amino acid sequence of SEQ ID NO: 2 or an amino acid sequence having at least 90% identity to SEQ ID NO: 2 can be used as the translocation peptide as recited herein. In one embodiment, an amino acid sequence of SEQ ID NO: 3 or an amino acid sequence having at least 90% identity to SEQ ID NO: 3 can be used as the linker peptide as recited herein.

Variants with sequence identity of the APC-binding peptide or the CD91 receptor-binding peptide, the translocation peptide, the cysteine-rich peptide, and the ER retention sequence can be used in the present disclosure. Methods of sequence alignment for comparison and determination of percent sequence identity constitute known technique. Optimal alignment of sequences for comparison can be conducted, e.g., by the homology alignment algorithm of Needleman and Wunsch, J. Mol. Biol. 48:443 (1970), by the search for similarity method of Pearson and Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, WI), by manual alignment and visual inspection (*see, e.g.,* Brent et al., Current Protocols in Molecular Biology (2003)), by use of algorithms know in the art including the BLAST and BLAST 2.0 algorithms, which are recited in Altschul et al., Nuc. Acids Res. 25:3389-3402 (1977); and Altschul et al., J. Mol. Biol. 215:403-410 (1990), respectively. Software for performing BLAST analyses is publicly available through the National Center for Biotechnology Information.

In one embodiment, in the cysteine-rich peptide carrying an antigen, the cysteine-rich peptide comprises the amino acid sequence of PCCGCCGCGC (SEQ ID NO: 4).

In one embodiment, any ER retention sequence can be used in the fusion protein. The endoplasmic reticulum (ER) retention sequence plays a vital role in controlling the surface expression of ion channels. It acts as a quality control mechanism, enabling only those ion channels that are properly assembled-wherein the retention signals are sterically concealed-to be transported to the cell surface. KDEL is a consensus sequence for ER retention. Accordingly, an ER retention sequence with KDEL-like sequence can be used in the present disclosure. In one embodiment of the fusion protein recited herein, the simultaneous presence of both the linker peptide (e.g., having the amino acid sequence of SEQ ID NO: 3) and the APC-presenting cell-binding peptide or the CD91 receptor-binding peptide (e.g., having the amino acid sequence of SEQ ID NO: 1) helps to stabilize the folding of the fusion protein and also promotes recognition of targets cells and stronger binding of receptors on the surface of target cells to the APC-presenting cell-binding peptide or the CD91 receptor-binding peptide with higher affinity, which in turn induces more efficient internalization of the fusion protein.

The details of the method for preparing a cysteine-rich peptide carrying an antigen via a linker are recited in Chiang, Hsiao-Ling et al. "A novel synthetic bipartite carrier protein for developing glycotope-based vaccines." Vaccine vol. 30,52 (2012): 7573-81. doi:10.1016/j.vaccine.2012.10.041.

In some embodiments, the fusion protein recited herein may include the peptide sequences shown in Table 1 in sequential order from the N-terminus to the C-terminus.

Table 1

| Peptide | SEQ ID NO | Sequence |
|---------|-----------|----------|
| PEA domain Ia | 1 | AEEAFDLWNECAKACVLDLKDGVRSSRMSVDPAIADT NGQGVLHYSMVLEGGNDALKLAIDNALSITSDGLTIRL EGGVEPNKPVRYSYTRQARGSWSLNWLVPIGHEKPSNI KVFIHELNAGNQLSHMSPIYTIEMGDELLAKLARDATF FVRAHESNEMQPTLAISHAGVSVVMAQTQPRREKRWS EWASGKVLCLLDPLDGVYNYLAQQRCNLDDTWEGKI YRVLAGNPAKHDLDIKPTVISHRLHFPE |
| PEA domain II | 2 | GGSLAALTAHQACHLPLETFTRHRQPRGWEQLEQCGY PVQRLVALYLAARLSWNQVDQVIRNALASPGSGGDLG EAIREQPEQARLALTLAAAESERFVRQGTGNDEAGAA N |
| PEA domain Ib | 3 | ADVVSLTCPVAAGECAGPADSGDALLERNYPTGAEFL GDG |
| Cysteine-rich peptide | 5 | PCCGCCGCGC PCCGCCGCGC PCCGCCGCGC PCCGCCGCGC PCCGCCGCGC PCCGCCGCGC |
| ER retention signal peptide | 8 | REDLK |

In addition, an antigen of interest, such as the cancer-associated glycoantigen recited herein, is linked to the cysteine residues in the cysteine-rich peptide noted by the method recited in detail in Chiang, Hsiao-Ling et al. "A novel synthetic bipartite carrier protein for developing glycotope-based vaccines." Vaccine vol. 30, 52 (2012): 7573-81. doi:10.1016/j.vaccine.2012.10.041. As an example, m-maleimidobenzoyl-N-hydroxysuccinimide ester (MBS) may be used as a cross-linker for conjugation of the antigen with the cysteine residues in the cysteine-rich peptide.

In some embodiments, the fusion protein recited herein includes or consists of the following sequence.

MAEEAFDLWNECAKACVLDLKDGVRSSRMSVDPAIADTNGQGVLHYSMVLEGGNDA

LKLAIDNALSITSDGLTIRLEGGVEPNKPVRYSYTRQARGSWSLNWLVPIGHEKPSNIKV

FIHELNAGNQLSHMSPIYTIEMGDELLAKLARDATFFVRAHESNEMQPTLAISHAGVSVV

MAQTQPRREKRWSEWASGKVLCLLDPLDGVYNYLAQQRCNLDDTWEGKIYRVLAGN

PAKHDLDIKPTVISHRLHFPEGGSLAALTAHQACHLPLETFTRHRQPRGWEQLEQCGYP

VQRLVALYLAARLSWNQVDQVIRNALASPGSGGDLGEAIREQPEQARLALTLAAAESE

RFVRQGTGNDEAGAANADVVSLTCPVAAGECAGPADSGDALLERNYPTGAEFLGDGE

LGGGGSGSPCCGCCGCGCPCCGCCGCGCPCCGCCGCGCPCCGCCGCGCPCCGCCGCGC

PCCGCCGCGCPCCGCCGCGCKLAAASGHHHHHHGREDLK (SEQ ID NO: 9).

By designing the fusion protein recited herein to include *Pseudomonas* exotoxin A domains Ia, II, Ib, a cysteine-rich peptide carrying an antigen (e.g., Tn antigen, T antigen, and/or sTn antigen), and a ER retention peptide, strong adaptive immune response including both humoral immune response and cell-mediated immune response may be successfully induced in a subject with cancer, thereby reducing the risk of cancer metastasis and recurrence for the subject.

*Pharmaceutical Composition and Administration Methods*

The fusion protein and the compositions as recited herein can be useful as a vaccine and/or antigenic compositions for inducing a protective immune response in a vertebrate. The disclosure provides pharmaceutical compositions comprising the fusion protein. The pharmaceutical compositions of the disclosure are formulated with suitable diluents, carriers, excipients, and other agents that provide improved transfer, delivery, tolerance, and the like. The compositions may be formulated for specific uses, such as for veterinary uses or pharmaceutical uses in humans. The form of the composition and the excipients, diluents, and/or carriers used will depend upon the intended use of the fusion protein and, for therapeutic use, the mode of administration. A multitude of appropriate formulations can be found in the formulary known to all pharmaceutical chemists, including Remington's Pharmaceutical Sciences, Mack Publishing Company, Easton, Pa. These formulations include, for example, powders, pastes, ointments, jellies, waxes, oils, lipids, lipid (cationic or anionic) containing vesicles (such as LIPOFECTIN.TM., Life Technologies, Carlsbad, Calif.), DNA conjugates, anhydrous absorption pastes, oil-in-water and water-in-oil emulsions, emulsions carbowax (polyethylene glycols of various molecular weights), semi-solid gels, and semi-solid mixtures containing carbowax. See also Powell et al. "Compendium of excipients for parenteral formulations" PDA (1998) J Pharm Sci Technol 52:238-311.

The fusion proteins and pharmaceutical compositions recited herein can be administered to a patient (e.g., a human patient suffering from cancer in a variety of dosage forms) to treat a cancer or induce an immune response.

Examples of cancer include, but are not limited to, breast cancer, liver cancer, colorectal cancer, bladder cancer, cervical cancer, leukemia, lung cancer, central nervous system cancer, melanoma, ovarian cancer, gastric cancer, pancreatic cancer, head and neck cancer, renal cancer, and prostate cancer.

The cancer can be treated via immune response, preferably an adaptive immune response. Examples of the adaptive immune response include, but are not limited to, a humoral immune response, a cell-mediated immune response, or a combination thereof.

The fusion protein and the compositions as recited herein may be administered by a variety of routes, such as orally, transdermally, subcutaneously, intranasally, intravenously, intramuscularly, intraocularly, or parenterally. The most suitable route for administration in any given case will depend on the fusion protein administered, the patient, pharmaceutical formulation methods, administration methods, the patient's age, body weight, sex, severity of the diseases being treated, the patient's diet, and the patient's excretion rate.

The effective amount of a particular agent may be represented in a variety of ways based on the nature of the agent, such as mass/volume, # of cells/volume, particles/volume, (mass of the agent)/(mass of the subject), # of cells/(mass of subject), or particles/(mass of subject). The effective amount of a particular agent may also be expressed as the half-maximal effective concentration ($EC_{50}$), which refers to the concentration of an agent that results in a magnitude of a particular physiological response that is halfway between a reference level and a maximum response level.

The following Examples are provided to elucidate certain aspects of the present invention and to aid those skilled in the art in practicing this invention. These Examples are in no way to be considered to limit the scope of the invention in any manner. Without further elaboration, it is believed that one skilled in the art can, based on the description herein, utilize the present invention to its fullest extent. All publications cited herein are hereby incorporated by reference in their entirety.

**EXAMPLES**

**Example 1: Induction of Cell-Mediated Immune Response by Fusion Protein**

To evaluate whether the fusion protein is capable of being internalized into the cytoplasm, mStrawberry (mSB) red fluorescence, as an indicator, was fused with the fusion protein bearing or lacking the PE domains (domains Ia, Ib, and II) and the ER retention sequence REDLK. The recombinant mSB fluorescence proteins were incubated with NIH 3T3 cells for 30 min at 37°C. Subsequently, the cells were washed with acidic PBS to remove excess recombinant fluorescence proteins. Images were acquired with an Olympus microscope equipped with an Olympus DP70 digital camera; Original magnification 10x; scale bar = 200$\mu$m.

As shown in **FIG. 2,** with the PE domains and the ER retention sequence REDLK, the mSB fluorescence proteins were able to enter the cells. In contrast, no fluorescence was observed in the group using the construct lacking the PE domains and the ER retention sequence REDLK. These results indicate that the importance of the PE domains and the ER retention sequence REDLK in internalization of the antigen into the cells.

**Example 2: Cell-Mediated Immunity Induced by Fusion Protein**

An LDH release assay was performed to confirm the ability of the fusion protein to induce enhanced cytotoxicity of T cells. The fusion protein (PE ($\triangle$III)+$Cys_{42}$Tn+REDLK) or an adjuvant (CpG1018) was injected intramuscularly to immunize BALB/c mice, once every two weeks for a total of four times (on day 1, day 15, day 29, and day 43). In particular, for the mice receiving the fusion protein, 50 $\mu$L of the fusion protein (with a concentration of 0.4 mg/mL) with the CpG1018 adjuvant was administered each time. As a control group, the mice in the other group received the CpG1018 adjuvant alone.

On day 49 from the immunization, T cells (effector cells, E) were isolated from the spleen, and were co-cultured with MDA-MB-231 cells expressing Tn antigen (target cells, T) at different T:E ratios for 18 hr at 37°C. In particular, 50 $\mu$L of cell suspension (MDA-MB-231 cells) was added to each well of a flat-bottom 96-well culture plate to achieve 6250 cells per well. 50 $\mu$L of T cell suspension (isolated from the mouse spleen) was added with different T:E ratios (T: MDA-MB-231 cells; E: T cells; T:E = 1:3, 1:10, 1:30, and 1:100). The co-cultures were incubated for 18 hr at 37°C. The amount of LDH released from damaged cells was then detected to determine cytotoxicity of T cells retrieved from mice treated with the fusion protein.

The percentage of cytotoxicity was calculated based on the following equation.

$$\underline{Cytotoxicity\ (\%) = (Test\ Group - Low\ Control)/(High\ Control - Low\ Control) * 100}$$

*Test Group: LDH release from MDA-MB-231 cells treated with the fusion protein*
*High Control: Total LDH in MDA-MB-231 cells without the treatment with the fusion protein*
*Low Control: Spontaneous LDH release from MDA-MB-231 cells without the treatment with the fusion protein*

The data in **FIG. 3** shows that the T:E ratio of 1: 100 resulted in a significant increase in cytotoxicity of T cells from the mice receiving the fusion protein against MDA-MB-231 cells expressing Tn antigen compared with that from the mice receiving the adjuvant alone ($p$=0.004), suggesting that the fusion protein is capable of inducing strong cell-mediated immune response.

## Example 3: Humoral Immune Response Induced by Fusion Protein

To evaluate whether the fusion protein is also able to induce humoral immune response, serum was collected from the mice in Example 2, which received the fusion protein or adjuvant, for monitoring production of the antibody against Tn antigen. Poly cysteine or poly cysteine conjugated with Tn were used as substrates to detect serum samples retrieved from mice immunized with the fusion protein or the adjuvant alone.

As evidenced by the Western blot assay shown in **FIG. 4,** antibodies in serum from the mice receiving the fusion protein were capable of specially recognizing Tn antigen. However, none of the serum samples from the adjuvant groups generated signals. This data shows that BALB/c after immunization with the fusion protein can produce humoral immunity as well.

Various references such as patents, patent applications, and publications are cited herein, the disclosures of which are hereby incorporated herein by reference in their entirety. Also, all references mentioned herein are specifically incorporated by reference to disclose and recite the methods and/or materials in connection with which the publications are cited.

## Claims

1. A fusion protein, comprising in sequential order from the N-terminus to the C-terminus:

   an antigen-presenting cell (APC)-binding peptide or a CD91 receptor-binding peptide having the amino acid sequence of SEQ ID NO: 1 or an amino acid sequence having at least 90% identity to SEQ ID NO: 1;
   a translocation peptide having the amino acid sequence of SEQ ID NO: 2 or an amino acid sequence having at least 90% identity to SEQ ID NO: 2;
   a peptide having the amino acid sequence of SEQ ID NO: 3 or an amino acid sequence having at least 90% identity to SEQ ID NO: 3;
   one or more repeats of cysteine-rich peptide carrying an antigen, wherein the cysteine-rich peptide comprises the amino acid sequence of PCCGCCGCGC (SEQ ID NO: 4); and
   optionally a retention signaling domain selected from an endoplasmic reticulum (ER) retention sequence, a Golgi retention sequence and a proteosome localizing sequence.

2. The fusion protein of Claim 1, wherein the cysteine-rich peptide comprises 2 to 15 repeats of the amino acid sequence of PCCGCCGCGC (SEQ ID NO: 4), optionally wherein the cysteine-rich peptide comprises 7 repeats of the amino acid sequence of PCCGCCGCGC (SEQ ID NO: 4).

3. The fusion protein of any one of the preceding claims, wherein the antigen is linked to at least one cysteine residue in the cysteine-rich peptide.

4. The fusion protein of any one of the preceding claims, wherein the antigen is a glycoantigen, or wherein the antigen comprises a cancer-associated antigen.

5. The fusion protein of any one of the preceding claims, wherein the antigen comprises one or more antigen selected from the group consisting of T, sTn, Tn, GM2, GM3, phosphoserine, phosphothreonine, sialic acid, GlcNAc, Globo H, Lewis x, Lewis y, and a steroid hormone, optionally wherein the antigen comprises a T antigen, an sTn antigen, a Tn antigen, or a combination thereof.

6. The fusion protein of any one of the preceding claims, wherein the ER retention sequence comprises the amino acid sequence of KDEL (SEQ ID NO: 6), RDEL (SEQ ID NO: 7), or REDLK (SEQ ID NO: 8), optionally wherein the ER retention sequence comprises the amino acid sequence of REDLK (SEQ ID NO: 8).

7. The fusion protein of any one of the preceding claims, wherein the fusion protein comprises in sequential order from the N-terminus to the C-terminus:

   the APC-binding peptide or the CD91 receptor-binding peptide;
   the translocation peptide;
   the peptide;
   a cysteine-rich peptide; and
   the ER retention sequence.

8. The fusion protein of any one of the preceding claims, wherein the fusion protein comprises the amino acid sequence of SEQ ID NO: 9.

9. An isolated nucleic acid, which encodes the fusion protein of any one of the preceding claims.

10. A host cell, comprising the isolated nucleic acid of Claim 9.

11. A pharmaceutical composition, comprising the fusion protein of any one of Claims 1 to 8 and a pharmaceutically acceptable carrier.

12. A method of treating cancer in a subject in need thereof, comprising administering to the subject an effective amount of the fusion protein of any one of Claims 1 to 8 or the pharmaceutical composition or Claim 11.

13. The method of Claim 12, wherein the cancer is selected from the group consisting of breast cancer, liver cancer, colorectal cancer, bladder cancer, cervical cancer, leukemia, lung cancer, central nervous system cancer, melanoma, ovarian cancer, gastric cancer, pancreatic cancer, head and neck cancer, renal cancer, and prostate cancer.

14. A method of inducing an immune response in a subject, comprising administering to the subject an effective amount of the fusion protein of Claims 1 to 8 or the pharmaceutical composition or Claim 11.

15. The method of Claim 14, wherein the immune response is an adaptive immune response, optionally wherein the adaptive immune response is a humoral immune response, a cell-mediated immune response, or a combination thereof.

FIG. 1A

FIG. 1B

FIG. 1C

FIG. 2

FIG. 3

FIG. 4

EP 4 595 975 A1

Europäisches
Patentamt

European
Patent Office

Office européen
des brevets

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2018/111964 A1 (LIAO CHAO-WEI [TW] ET AL) 26 April 2018 (2018-04-26) * the whole document; in particular paragraphs 39, 62; tables 17, 18; paragraphs 79-106; SEQ ID NOs: 32-43 * | 1-15 | INV. A61K39/00 A61P35/00 C07K7/06 C07K14/21 |
| A | WEN-FANG CHENG ET AL: "Fusion Protein Vaccines Targeting Two Tumor Antigens Generate Synergistic Anti-Tumor Effects", PLOS ONE, vol. 8, no. 9, 1 September 2013 (2013-09-01), pages e71216-1, XP055605469, DOI: 10.1371/journal.pone.0071216 * the whole document; in particular figure 1 * | 1-15 | |
| A | LIAO CHAO-WEI ET AL: "Fusion protein vaccine by domains of bacterial exotoxin linked with a tumor antigen generates potent immunologic responses and antitumor effects", CANCER RESEARCH, UNIVERSITY OF CHICAGO PRESS, vol. 65, no. 19, 1 October 2005 (2005-10-01), pages 9089-9098, XP002374092, ISSN: 0008-5472, DOI: 10.1158/0008-5472.CAN-05-0958 * the whole document * | 1-15 | TECHNICAL FIELDS SEARCHED (IPC) C07K A61K A61P |

-/--

The present search report has been drawn up for all claims

| Place of search | Date of completion of the search | Examiner |
|---|---|---|
| Munich | 13 June 2025 | Stein, Annette |

Europäisches
Patentamt

European
Patent Office

Office européen
des brevets

# EUROPEAN SEARCH REPORT

**Application Number**

EP 25 15 3235

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | HSIAO-LING CHIANG ET AL: "A novel synthetic bipartite carrier protein for developing glycotope-based vaccines", VACCINE, vol. 30, no. 52, 1 December 2012 (2012-12-01), pages 7573-7581, XP055255756, AMSTERDAM, NL ISSN: 0264-410X, DOI: 10.1016/j.vaccine.2012.10.041 * the whole document; in particular figure 2; page 7575-7577, bridging paragraph and discussion * | 1-15 | |
| Y | US 2009/324619 A1 (HWANG JAULANG [TW] ET AL) 31 December 2009 (2009-12-31) * paragraphs [0005] - [0008], [0017], [0020] - [0023]; figure 1; examples 1-4 * | 1-15 | |

TECHNICAL FIELDS
SEARCHED    (IPC)

The present search report has been drawn up for all claims

| Place of search | Date of completion of the search | Examiner |
|---|---|---|
| Munich | 13 June 2025 | Stein, Annette |

EPO FORM 1503 03.82 (P04C01)

**ANNEX TO THE EUROPEAN SEARCH REPORT
ON EUROPEAN PATENT APPLICATION NO.**

EP 25 15 3235

This annex lists the patent family members relating to the patent documents cited in  the above-mentioned European search report.
The members are as contained in the European Patent Office EDP file on
The European Patent Office is in no way liable for these particulars which are merely given for the purpose of information.

13-06-2025

| Patent document cited in search report | Publication date | Patent family member(s) | | Publication date |
|---|---|---|---|---|
| US 2018111964 A1 | 26-04-2018 | TW | 201829470 A | 16-08-2018 |
| | | US | 2018111964 A1 | 26-04-2018 |
| | | WO | 2018081355 A1 | 03-05-2018 |
| US 2009324619 A1 | 31-12-2009 | TW | 201006490 A | 16-02-2010 |
| | | US | 2009324619 A1 | 31-12-2009 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 63623781

### Non-patent literature cited in the description

- **NEEDLEMAN** ; **WUNSCH**. *J. Mol. Biol.*, 1970, vol. 48, 443
- **PEARSON** ; **LIPMAN**. *Proc. Nat'l. Acad. Sci. USA*, 1988, vol. 85, 2444
- **BRENT et al.** *Current Protocols in Molecular Biology*, 2003
- **ALTSCHUL et al.** *Nuc. Acids Res.*, 1977, vol. 25, 3389-3402
- **ALTSCHUL et al.** *J. Mol. Biol.*, 1990, vol. 215, 403-410
- **CHIANG, HSIAO-LING et al.** A novel synthetic bipartite carrier protein for developing glycotope-based vaccines. *Vaccine*, 2012, vol. 30 (52), 7573-81
- Remington's Pharmaceutical Sciences. Mack Publishing Company
- **POWELL et al.** Compendium of excipients for parenteral formulations. *J Pharm Sci Technol*, 1998, vol. 52, 238-311